# FASCICULE DE BREVET EUROPEEN

(11) **EP 3 212 094 B1**
(45) Date de publication et mention de la délivrance du brevet: **12.12.2018**
(21) Numéro de dépôt: 15790081.2
(22) Date de dépôt: 29.10.2015
(51) Int. Cl.: A61B 17/06

(54) **BASE SUPPORT AMBIDEXTRE POUR FIL DE SUTURE**
BEIDHÄNDIGER TRÄGER FÜR EINEN NAHTFADEN
AMBIDEXTROUS SUPPORT BASE FOR A SUTURE THREAD

(30) Priorité: 29.10.2014 FR 1460408; 25.02.2015 FR 1551626
(43) Date de publication de la demande: 06.09.2017
(73) Titulaire: Peters Surgical, 93000 Bobigny (FR)
(72) Inventeur: HALFON, Thomas, F-93460 Gournay sur Marne (FR); CAMEDDA, Caroline, F-95410 Groslay (FR)
(74) Mandataire: Regimbeau
(86) Numéro de dépôt international: PCT/EP2015/075170
(87) Numéro de publication internationale: WO 2016/066767

(56) Documents cités:
- EP-A1- 0 082 218
- EP-A1- 1 275 343
- EP-A2- 1 106 142
- US-A- 5 906 273
- US-A1- 2007 227 914
- US-A1- 2007 256 945

## Description

### DOMAINE GENERAL

L'invention se rapporte au domaine du conditionnement de matériel chirurgical.

L'invention concerne plus particulièrement une partie d'emballage formant une base support pour fil de suture.

### ETAT DE L'ART

On connait de l'état de l'art divers types d'emballage pour fil de suture.

Un premier type d'emballage connu depuis de très nombreuses années consiste en une pochette ou blister, déchirable ou pelable, pouvant lui-même contenir une deuxième pochette ou blister, qui loge une navette support dans laquelle est enroulé le fil de suture. On connaît des navettes formées d'un simple cartonnage sur lequel le fil de suture est enroulé en O ou en 8.

On connaît également des navettes comprenant une base présentant deux côtés opposés, la base comprenant sur un de ses côtés, un canal de retenue d'un fil de suture dans une position enroulée, et une zone de parcage d'une extrémité du fil de suture munie d'une aiguille ou deux, la zone de parcage étant entourée par le canal de retenue. Le canal de retenue permet ainsi d'éviter la formation de noeuds. Pour extraire le fil, un opérateur saisit d'une main la navette, saisit de l'autre main l'extrémité de ce fil localisée dans la zone de parcage à l'aide d'un porte aiguille, et dévide ainsi le fil hors du canal qui le contenait. Par convention, on considérera dans la suite que la préhension de l'extrémité du fil de suture signifie la préhension de l'aiguille lorsqu'elle est présente, l'aiguille étant à l'extrémité du fil de la suture.

Toutefois, le fil de suture n'est accessible que depuis l'un des deux côtés de la base. En conséquence, le fil de suture contenu dans une telle navette n'est pas aussi facile à extraire selon que l'opérateur saisit l'extrémité du fil par la main droite ou par la main gauche.

Pour remédier à ce problème, il a été proposé dans le document US 5,906,273, comme illustré en **figure 1** annexée, une navette E qui se veut ambidextre et comprenant, en sus des caractéristiques précitées, une fenêtre traversante O entre les deux côtés de la base, la fenêtre O étant ménagée dans la zone de parcage.

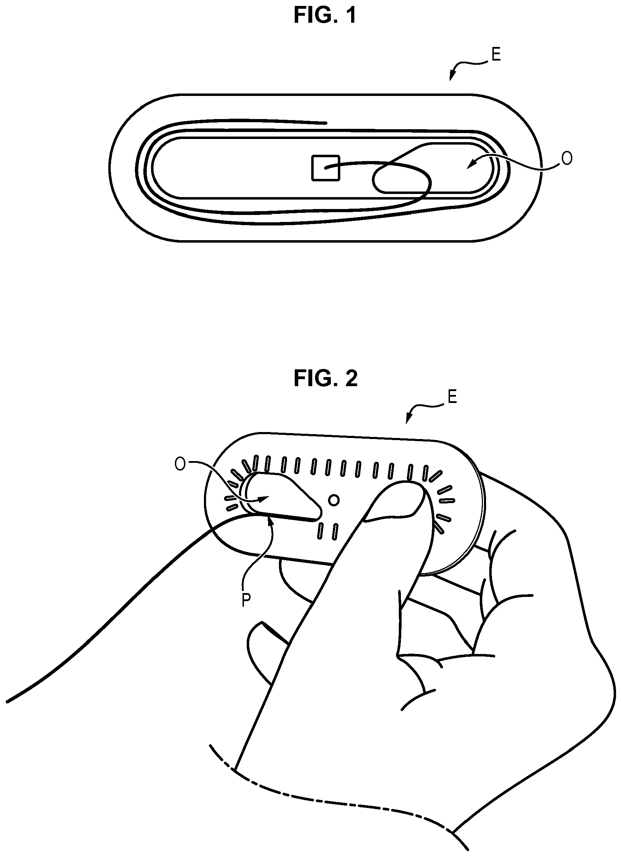

Un opérateur peut saisir l'extrémité du fil de suture depuis chacun des deux côtés de la base, en utilisant l'une ou l'autre de ses mains. Dans une première utilisation, l'opérateur peut en effet saisir avec une première main (par exemple la droite) l'extrémité du fil depuis le côté sur lequel s'étend le canal de retenue et la zone de parcage ; et dans une deuxième utilisation alternative, l'opérateur peut saisir l'extrémité de fil localisée dans la zone de parcage insérée depuis le côté opposé de la base au moyen de sa deuxième main (ici la gauche). Le fil de suture va alors, au cours de son extraction, passer à travers la fenêtre traversante, comme illustré en **figure 2****.**

Cependant, le geste selon cette deuxième utilisation est complexe.

Par ailleurs, au cours de l'extraction du fil dans cette deuxième utilisation, le fil de suture est exposé à des frottements qui ne sont pas rencontrés dans la première utilisation. Comme le canal de retenue entoure la fenêtre traversante, le fil en cours d'extraction se tend et former un angle aigu en un bord intérieur du canal de retenue, ce qui est une première source de frottement, et éventuellement sur un bord de la fenêtre traversante, deuxième source de frottement, au niveau du point P illustré sur la figure 2. De tels frottements peuvent non seulement abîmer le fil de suture, généralement de faible diamètre, mais également conduire à des résistances rendant plus difficile l'extraction.

Il a également été proposé dans le document EP 1 106 142 A2 une base support comprenant une zone de parcage périphérique dans laquelle est formée une ouverture traversante.

### PRESENTATION DE L'INVENTION

L'invention vise donc à proposer un emballage pour fil de suture hors duquel le fil de suture peut être extrait aussi aisément par la main droite que par la main gauche, sans risquer d'endommager ou de coincer le fil de suture par l'une ou l'autre de ces deux utilisations.

Dans ce but, il est proposé une base support pour fil de suture présentant deux côtés opposés et comprenant sur un de ses côtés un canal de retenue d'un fil de suture dans une position enroulée, et une zone de parcage d'une extrémité du fil de suture, la zone de parcage étant entourée par le canal de retenue, la base support étant caractérisée en ce qu'elle présente une ouverture traversante entre ses deux côtés, l'ouverture traversante s'étendant dans la zone de parcage et se prolongeant depuis la zone de parcage de façon à couper le canal de retenue. La base support comprend en outre un volet mobile entre une position ouverte pour dévoiler l'ouverture traversante entre ses deux côtés, et une position fermée pour boucher au moins en partie l'ouverture traversante, le volet mobile étant configuré pour faire saillie, dans sa position ouverte, depuis le côté de la base opposé au côté sur lequel le canal de retenue est formé.

Lorsqu'un opérateur fait passer l'extrémité du fil de suture par l'ouverture traversante, et qu'il tire sur cette extrémité afin de dévider le fil de suture, ce fil va naturellement quitter la partie de l'ouverture localisée dans la zone de parcage et se tendre selon un axe présentant une déviation angulaire faible par rapport à la trajectoire d'enroulement du fil dans le canal de retenue, cette déviation étant imprimée dans la partie de l'ouverture traversante qui coupe le canal de retenue. L'opérateur peut ainsi, en éloignant ses deux mains l'une de l'autre, dévider le fil sans que le fil ne soit fortement contraint mécaniquement par un quelconque bord de l'ouverture traversante.

L'invention peut également être complétée par les caractéristiques suivantes, prises seules ou en une quelconque de leurs combinaisons techniquement possibles.

Le canal de retenue étant délimité extérieurement par une paroi périphérique constituant un bord libre de la base, l'ouverture traversante peut être prolongée jusqu'à interrompre également la paroi périphérique. Une telle extension de l'ouverture traversante permet de limiter les frottements sur le fil en cours d'extraction même lorsque l'opérateur éloigne ses deux mains l'une de l'autre dans un mouvement naturel en arc de cercle.

Le canal de retenue peut en outre comprendre un premier tronçon dont une première extrémité débouche dans l'ouverture traversante, l'extrémité du fil localisée dans la zone centrale de parcage s'échappant du canal de retenue par la première extrémité. Lorsque l'opérateur éloigne l'extrémité du fil de la base au cours de l'extraction, le fil se tend ainsi tout naturellement entre la première extrémité de tronçon et l'extrémité du fil sans manipulation complexe préalable.

Le premier tronçon peut être rectiligne suivant un axe longitudinal, de façon à offrir à l'opérateur un axe d'extraction préféré dans lequel les frottements sont minimisés.

La première extrémité peut présenter un bord de jonction reliant les deux côtés de la base, le bord de jonction s'étendant perpendiculairement à l'axe longitudinal. Si, lors de l'extraction du fil de suture hors de la base, l'opérateur effectue un mouvement naturel en arc de cercle, le fil de suture glisse progressivement le long du bord de jonction sans accroc.

Le canal de retenue peut comprendre en outre un deuxième tronçon dont une deuxième extrémité en regard de la première extrémité débouche également dans l'ouverture traversante, le deuxième tronçon s'étendant également suivant le même axe longitudinal. Ainsi, même si l'ouverture traversante interrompt localement le canal de retenue entre le premier tronçon et le deuxième tronçon, le fil de suture n'est pas dévié par la présence de cette ouverture, et le dévidage du fil peut être opéré sans frottement supplémentaire.

La première extrémité et la deuxième extrémité peuvent en outre être de mêmes largeurs entre la zone de parcage et la paroi périphérique de la base.

L'ouverture traversante peut s'étendre entre les deux extrémités de tronçon en regard sur une première longueur suivant l'axe longitudinal, et s'étendre dans la zone de parcage sur une deuxième longueur parallèle et supérieure à la première longueur. Ceci permet de faciliter la préhension de l'extrémité du fil de suture depuis le côté opposé à celui sur lequel la zone de parcage est formée, tout en évitant que des portions du fil de suture enroulé, accessibles entre les extrémités du premier tronçon et du deuxième tronçon en regard l'une de l'autre, ne glissent vers la zone de parcage, gênant ainsi la préhension de l'extrémité du fil de suture.

Le premier tronçon peut présenter une longueur suivant l'axe longitudinal supérieure à celle du deuxième tronçon. Le premier tronçon présente ainsi une plus grande surface de préhension à l'opérateur, et permet d'augmenter de la sorte le confort d'extraction.

Le canal de retenue peut par ailleurs être en forme sensiblement ellipsoïde. Une telle forme, dénuée d'angle aigu, permet d'évite des frottements à l'intérieur du canal au cours du dévidage du fil de suture et de minimiser la mémoire de forme du fil une fois dévidé.

La base support peut en outre être réalisée en un matériau rigide, plus facilement préhensible qu'un simple cartonnage, et/ou imperméable aux fluides, dont la fabrication permet des géométries plus variées.

La base support peut comprendre par ailleurs une nervure de rigidification faisant saillie depuis au moins un des deux côtés opposés de la base, la nervure de rigidification s'étendant le long d'un bord de l'ouverture traversante.

La nervure de rigidification peut s'étendre entre l'ouverture traversante et un tronçon du canal de retenue présentant globalement une forme en C.

Le volet mobile peut présenter une extrémité de liaison montée pivotante à une partie de la base solidaire de la paroi périphérique, et un bord libre opposé à l'extrémité de liaison et agencé pour être aligné avec ou dépassant de la paroi périphérique lorsque le volet mobile est positionné dans sa position fermée. Un tel bord libre permet ainsi de saisir facilement le volet mobile pour le déplacer de sa position fermée vers sa position ouverte.

Le volet mobile peut présenter deux côtés opposés formant chacun une portion des côtés opposés de la base lorsque le volet mobile est positionné dans sa position fermée. Ainsi, l'épaisseur de la base support reste faible malgré l'adjonction du volet mobile.

L'invention concerne également un procédé d'extraction d'un fil de suture enroulé dans le canal de retenue d'une base support du type précité, le procédé comprenant les étapes suivantes :
- insertion d'un moyen de préhension à travers l'ouverture traversante par le côté de la base opposé au côté sur lequel le canal de retenue est formé,
- saisie de l'extrémité du fil de suture parquée dans la zone de parcage à l'aide du moyen de préhension passé à travers l'ouverture traversante,
- éloignement mutuel de la base et de l'extrémité du fil de suture saisie par le moyen de préhension.

Dans le cas où la base comprend un volet mobile, le procédé ci-dessus peut comprendre une étape de déplacement du volet mobile (8) depuis sa position fermée dans sa position ouverte, avant l'étape d'insertion.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
- Les figures 1 et 2, déjà discutées, représentent un emballage connu pour fil de suture.
- Les figures 3 et 4 sont deux vues de face d'une base support pour fil de suture selon un premier mode de réalisation de l'invention.
- La figure 5 est une vue de dos de la base support déjà représentée sur les figures 2 et 3.
- Les figures 6 à 8 représentent la base support des figure 2 à 4 manipulé par un opérateur afin d'extraire le fil de suture qu'elle contient selon une première utilisation.
- La figure 9 représentent la base support de la figure 2 à 4 manipulé par un opérateur afin d'extraire le fil de suture qu'elle contient selon une deuxième utilisation.
- La figure 10 est une vue de face d'une base support pour fil de suture selon un deuxième mode de réalisation de l'invention.
- La figure 11 est une vue de trois quarts partielle du mode de réalisation de la figure 10.
- La figure 12 est une vue de dos de la base support représentée sur les figures 10 et 11.
- Les figures 13 à 15 sont des vues d'une base support selon un troisième mode de réalisation de l'invention.
Sur l'ensemble des figures, les éléments similaires portent des références identiques.

### DESCRIPTION DETAILLEE DE L'INVENTION

Les **figures 1 et 2** ont déjà été décrites.

Un emballage pour fil de suture comprend une enveloppe blister ou pochette (non illustrée), et une base support 1 sensiblement plane destinée à être dans l'enveloppe.

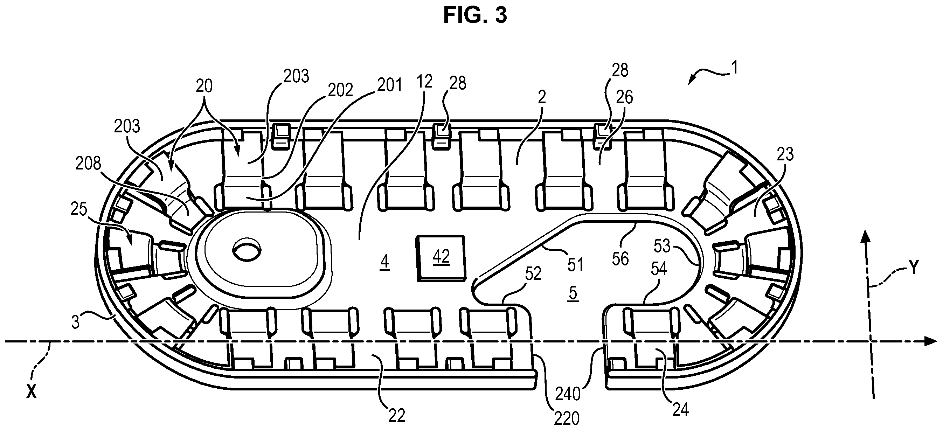

En référence aux **figures 3 à 5****,** la base support 1 comprend un premier côté 12 et un deuxième côté 14 opposé au premier côté 12.

La base support est de contour globalement ovale, ellipsoïde ou en hippodrome.

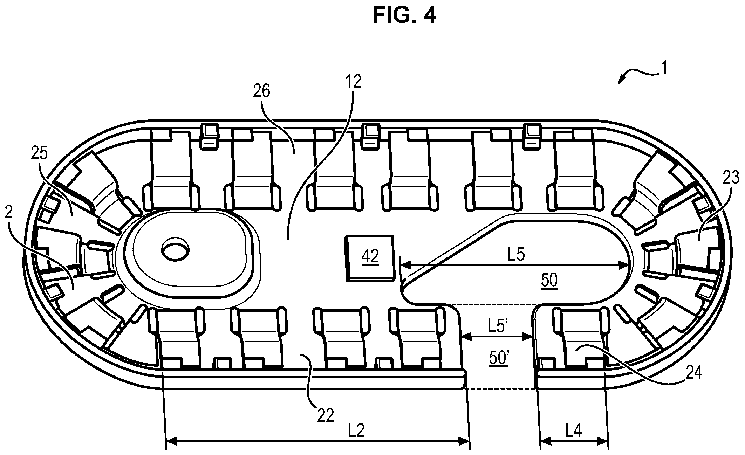

La surface du premier côté 12, visible sur les figures 3 et 4, est divisé en deux zones : une zone centrale de parcage 4, et une zone périphérique entourant la zone de parcage 4, zone périphérique sur laquelle est formé un canal de retenue 2 destiné à contenir un fil de suture.

Le canal de retenue 2 est défini par une paroi extérieure 3 périphérique et des moyens de retenue intérieurs, la paroi et lesdits moyens de retenue faisant saillie depuis le premier côté 12.

La paroi extérieure 3 présente une surface extérieure formant un bord libre de la base 1. Ce bord libre est préhensible par un opérateur.

Les moyens de retenue intérieurs délimitent la zone de parcage 4. Dans le mode de réalisation illustré sur les figures, ces moyens de retenue sont formés par une pluralité de doigts 20 faisant saillie depuis le côté 12 de la base 1.Les doigts 20 sont disposés selon une trajectoire en hippodrome ou ovale circonscrite par le bord libre de la base 1, de trajectoire similaire. Un fil de suture peut être logé dans le canal de retenue 2 selon une trajectoire similaire ou tout du moins une trajectoire ellipsoïdale.

Chaque doigt 20 présente une partie flexible 201 solidaire de la base et s'étendant dans le plan de la base. La partie flexible 201 est d'épaisseur adaptée pour servir de pivot élastique au doigt 20 correspondant. La partie flexible 201 est prolongée par une partie intermédiaire 202 formant un coude avec la partie flexible 201 de sorte à faire saillie depuis le premier côté 12, par exemple perpendiculairement au premier côté 12. La partie intermédiaire 202 est elle-même prolongée par une partie terminale 203 formant un coude avec la partie intermédiaire 202 et s'étendant en porte à faux vers le bord périphérique 3 dans un plan parallèle au plan de la base, afin de retenir un fil de suture logé entre la paroi extérieure du canal et la partie intermédiaire 202.

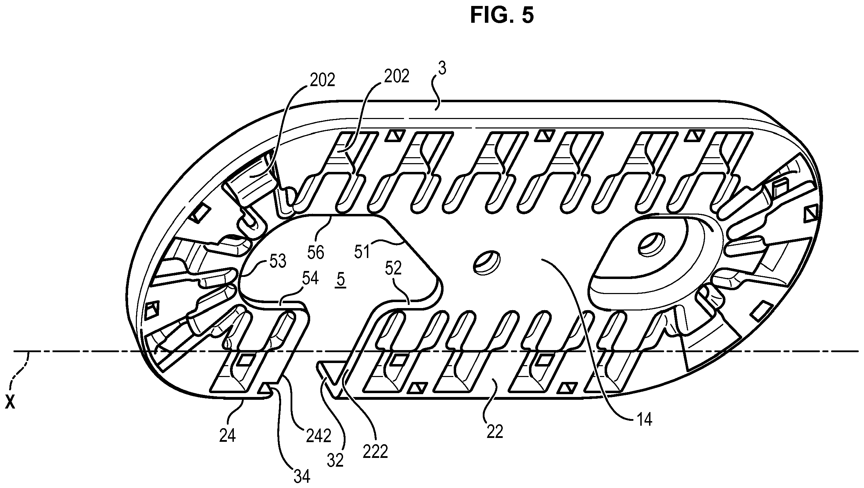

Le côté 14 de la base opposé au côté 12 est visible sur la figure 5. La base présente une pluralité de trous périphériques entre ses deux côtés 12 et 14 et accessibles depuis le côté 14. Chaque trou périphérique est ménagé entre le bord périphérique de la base et la partie flexible 201 d'un doigt 20 correspondant. Chaque partie terminale 203 surplombe le trou périphérique correspondant.

Chaque trou périphérique présente des dimensions légèrement plus grandes que la partie terminale qui le surplombe afin de permettre le démoulage de la pièce.

La base 1 présente en outre une ouverture traversante 5 entre ses deux côtés opposés 12 et 14. Cette ouverture traversante 5 présente une partie 50 s'étendant dans la zone centrale de parcage 4. La partie 50 est prolongée par une partie 50' qui s'étend dans la zone périphérique de façon à interrompre localement le canal de retenue 2 et couper la trajectoire d'enroulement du canal 2.

En conséquence, un fil de suture enroulé dans le canal de retenue 2 sera visible depuis chacun des deux côtés 12 et 14 de la base au niveau de la partie 50' de l'ouverture traversante 5.

La partie 50' de l'ouverture traversante 5 se prolonge en outre jusqu'à la paroi extérieure 3 de façon à l'interrompre localement. L'ouverture traversante forme ainsi une baie dans le plan de la base, la baie étant accessible depuis le bord libre de la base formé par la paroi extérieure 3.

Dans le mode de réalisation illustré sur les figures 3 à 5, le canal de retenue 2 comprend un premier tronçon 22 et un deuxième tronçon 24 s'étendant chacun le long d'un même axe longitudinal X, chaque tronçon 22, 24 se terminant par une extrémité respective 220, 240 du canal 2 qui débouche directement dans l'ouverture traversante 5.

Le canal de retenue 2 est constitué des tronçons successifs suivants depuis l'extrémité 220 : le premier tronçon rectiligne 22, un premier tronçon courbe 25 tournant à 180 degrés, un troisième tronçon rectiligne 26 parallèle au premier tronçon 22, puis un autre tronçon courbe 23 tournant à 180 degrés, puis enfin le deuxième tronçon 24 terminé par l'extrémité 240. Les deux tronçons courbes 23 et 25 sont symétriques et situés de part et d'autre de la zone de parcage 4.

Les tronçons courbes 23 et 25 présentent globalement une forme en C lorsque la forme générale de la base est en hippodrome. D'autres formes courbes pour les tronçons courbes peuvent cependant être prévues.

Les deux extrémités 220 et 240, séparées par la partie 50' de l'ouverture traversante 5 sont en regard l'une de l'autre et centrées sur le même axe longitudinal X de façon à permettre au fil de suture d'être enroulé dans le canal de retenue 2 selon une trajectoire ellipsoïde, dénuée de déviations brusques susceptible d'user localement le fil au moment de son dévidage.

Chaque extrémité 220, 240 présente un bord de jonction respectif 222, 242 reliant les deux côtés 12 et 14 de la base, et un bord extérieur respectif 32, 34 qui termine en outre la paroi extérieure 3. Le bord extérieur et le bord de jonction sont par exemple coplanaires, et le bord extérieur forme un coude à angle droit par rapport au bord de jonction. Au niveau de chaque extrémité 220, 240, la paire correspondante formée d'un bord de jonction et d'un bord extérieur est ainsi en forme de L.

Chaque bord de jonction 222, 242 est rectiligne, lisse et s'étend dans le plan de la base selon une direction transversale Y perpendiculaire à la direction de l'axe longitudinal X, entre le bord extérieur correspondant et la zone de parcage 4.

La partie 50 de l'ouverture traversante 5 qui est localisée dans la zone de parcage est délimitée par un contour dont une première extrémité se termine par le bord de jonction 220 et une deuxième extrémité se termine par le bord de jonction 240.

Le contour de la partie 50 de l'ouverture traversante 5 présente les portions successives suivantes depuis le bord de jonction 220 jusqu'au bord de jonction 240 : un premier bord 52 de retrait longeant le premier tronçon 22 dans la zone de parcage 4 parallèlement à l'axe longitudinal X, un bord de liaison 51 diagonal reliant le premier tronçon 22 au tronçon référencé 26, un bord rectiligne 56 longeant le tronçon 26, un bord courbe 53 longeant le tronçon courbe 23, et un deuxième bord 54 de retrait longeant le deuxième tronçon 24 dans la zone de parcage 4 parallèlement à l'axe longitudinal X.

L'ouverture traversante 5 ainsi délimitée occupe une portion extrémale de la zone de parcage 4, cette portion étant plus proche du tronçon courbe 23 que du tronçon courbe 25 qui lui est opposé.

Sur la portion de surface du côté 12 située dans la zone de parcage 4, et plus précisément entre le bord de liaison 51 et le tronçon courbe 25, est fixé un support 42 pour aiguille. Un tel support 42 se présente généralement sous la forme d'une mousse dans laquelle une aiguille peut être piquée et hors de laquelle l'aiguille peut être facilement retirée. Le support 42 est positionné dans la zone de parcage 4 de façon à ce qu'une aiguille piquée dans ce support 42 puisse surplomber partiellement la partie 50 de l'ouverture traversante 5 (typiquement 1/3 de l'aiguille). Ainsi, pour une grande aiguille, le support 42 peut être éloigné du bord 51.

Le bord de liaison 51 diagonal s'étend rectilignement selon une direction sécante à l'axe longitudinal X, le bord de liaison diagonal 51 s'éloignant du tronçon 26 depuis le bord 56 vers le bord 52. Cette direction sécante forme un angle typiquement compris entre 20° et 60° par rapport à la direction de l'axe longitudinal, dans le plan de la base 1.

Une telle orientation est adaptée à des aiguilles courbes de longueurs variables, couramment utilisées pour des fils de suture. Une aiguille arquée peut en effet être piquée dans le support 42 de façon à être contenu dans un plan parallèle à celui de la base 1, et à surplomber la partie 50 de l'ouverture traversante 5, facilitant ainsi sa préhension comme le verra dans la suite.

La base support 1 peut également comprendre des moyens d'accrochage d'une étiquette cartonnée au-dessus du côté 12, l'étiquette pouvant par exemple comprendre des inscriptions.

Ces moyens d'accrochage comprennent par exemple une pluralité de pions 28 faisant saillie depuis la paroi périphérique vers la zone de parcage 4, ces pions étant localisés à une hauteur légèrement supérieure à la hauteur occupée par la face supérieure des portion 203 des doigts de retenue 20. Une étiquette cartonnée présentant des dimensions adaptées au pourtour intérieur de la paroi 3 peut ainsi être coincée entre la pluralité de pions 28 et la pluralité de doigts 20.

La zone de parcage 4 peut également comprendre une portion localement surélevée par rapport au reste de la zone de parcage, cette portion surélevée étant destinée à servir de support pour l'étiquette cartonnée. Cette portion localement surélevée offre également une zone de préhension pour un utilisateur.

### Insertion du fil de suture dans la base support

La base support décrite précédemment peut contenir différents types de fil de suture :
- un fil comprenant une aiguille sertie à l'une de ses deux extrémités ;
- un fil comprenant deux aiguilles serties à ses deux extrémités opposées ;
- un fil comprenant une unique aiguille sertie à ses deux extrémités formant une boucle. Dans ce cas, les deux extrémités du fil se rejoignent dans l'aiguille ; ou bien
- un ou plusieurs fils nus appelés « brins ».

Dans la suite, on prendra l'exemple d'un fil pourvu d'une aiguille sertie à l'une de ses extrémités. Par convention, on considéra également que l'aiguille constitue elle-même l'extrémité du fil de la suture ; aussi, on comprendra que la préhension de l'extrémité du fil de suture signifie la préhension de l'aiguille.

Le fil de suture peut être logé dans la base support 1 de la façon suivante.

Le côté 14 de la base 1 est posé sur un support plan présentant une pluralité de pions en saillie répartis de façon à ce que chaque pion loge partiellement dans un trou correspondant laissé au niveau d'un doigt 20.

En plaquant la base 1 sur le support plan, chaque pion exerce une pression sur la partie terminale 203 d'un doigt 20. Les doigts 20 se lèvent simultanément au niveau de leur partie flexible 201 et 202. Le canal de retenue 2 est alors accessible depuis le côté 12 sur une trajectoire ovoïde formant une ligne fermée délimitée intérieurement par les parties intermédiaires 202 des différents doigts 20.

L'aiguille est piquée dans le support 42 située dans la zone de parcage 4 de la base 1.

Par ailleurs, le fil de suture est enroulé sur un ou plusieurs tours autour des parties intermédiaires 202 de la pluralité de doigts 20, à commencer par le doigt du premier tronçon 22 qui est le plus proche de l'extrémité 220.

Lorsque la base 1 est retirée du support, après que le fil a été enroulé, chaque doigt 20 reprend par retour élastique sa position de repos dans laquelle sa partie terminale 203 s'étend parallèlement au plan de la base, retenant ainsi une portion du fil enroulé.

Le fil de suture est alors logé dans la base 1. Le canal de retenue 2 permet d'éviter la formation de noeuds susceptibles de gêner son extraction hors de la base 1.

A ce stade, le fil de suture présente ainsi une portion terminale (coté aiguille) non contenue dans le canal de retenue 2. Cette portion de fil non enroulée s'échappe en effet de la première extrémité 220 du tronçon 22, s'étend dans la zone de parcage en surplombant l'ouverture traversante 5, et se termine par l'aiguille partiellement piquée dans le support 42.

### Extraction du fil de suture hors de la base support

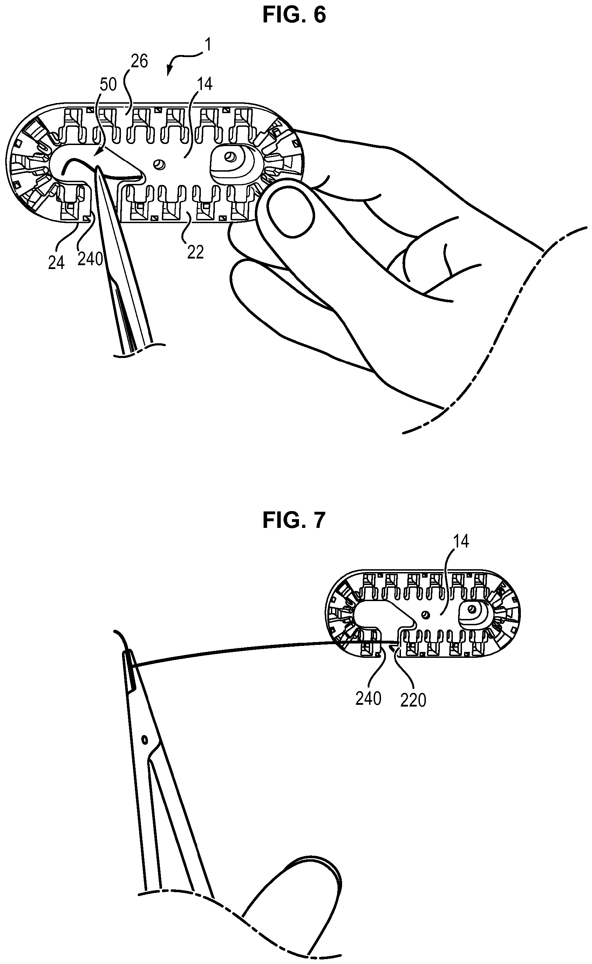

On va maintenant décrire comment le fil de suture peut être extrait de la base support 1 selon une première utilisation en regard des **figures 6 à 8****.**

Un opérateur pince la base 1 entre les doigts de sa main droite, de façon à ce que le côté 14 lui fasse face. Le fil et l'aiguille n'est donc pas entièrement visible. Il peut par exemple saisir la paroi extérieure au niveau du tronçon 22 par le pouce, et cette même paroi extérieure au niveau du tronçon 26 par l'index.

L'opérateur tient dans sa main gauche un instrument de préhension tel qu'une pince. L'opérateur fait alors passer depuis le côté 14 de la base 1 les mâchoires de la pince par l'ouverture traversante 5 au niveau de sa partie 50. En traversant l'ouverture traversante 5, les mâchoires de la pince pénètrent dans la zone de parcage 4 et saisissent la partie non-enroulée du fil de suture qui surplombe l'ouverture traversante 5 (par l'aiguille, qui offre une meilleure prise que le fil lui-même).

L'opérateur dégage l'aiguille du support mousse 42 puis fait ensuite traverser l'aiguille ainsi saisie dans l'ouverture traversante 5 depuis le côté 12 vers le côté 14. L'aiguille pénètre alors dans le demi-plan de l'espace défini par le côté 14 de la base 1 faisant face à l'opérateur.

L'opérateur éloigne ensuite la pince, généralement appelé « porte-aiguille », de la base 1 selon le mouvement représenté sur la figure 7. Ce faisant, la portion de fil non-enroulée se tend progressivement entre la première extrémité 220 depuis laquelle elle s'échappe, et les mors du porte-aiguille de façon à ce que cette portion de fil non enroulée effleure le bord de jonction 240 contre le côté 14 et décrive une droite sensiblement parallèle à l'axe longitudinal X du premier tronçon 22.

A partir du moment où la portion du fil de suture non enroulé est parfaitement tendu, la portion du fil enroulée dans le canal de retenue se dévide progressivement, et ce sans déviation importante susceptible de provoquer des frottements et/ou pincements importants sur le fil.

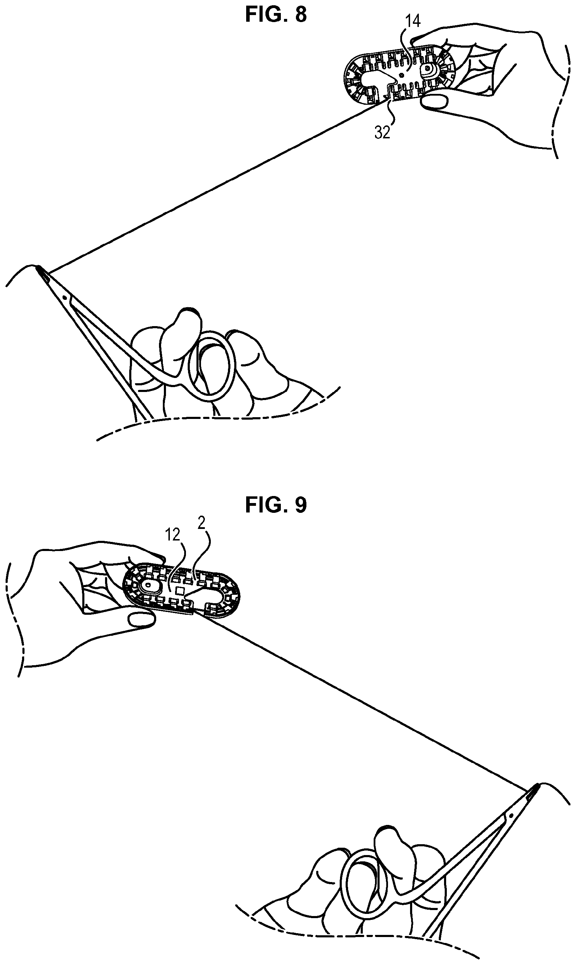

A fur et à mesure que l'opérateur continue à écarter ses deux mains l'une de l'autre, l'aiguille saisie suit naturellement une trajectoire en arc de cercle, comme représenté sur la figure 8, et le fil enroulé se dévide. Dans ce mouvement, le fil de suture en cours de dévidage va alors longer progressivement le bord de jonction 220 en direction du bord extérieur 32 et jusqu'à toucher ce dernier, sans toutefois imposer de contrainte mécanique importante au fil. Le fil décrit alors un angle relativement faible et contrôlable par l'opérateur en ce bord extérieur 32 jusqu'à ce que le fil de suture soit entièrement extrait hors de la base 1.

Selon une deuxième utilisation alternative illustrée en figure 9, l'opérateur saisit l'emballage par sa main gauche, de sorte que le côté 12 sur lequel la mousse 42 est fixée lui fasse face. Il peut par exemple saisir la paroi extérieure au niveau du tronçon 22 par le pouce, et cette même paroi extérieure au niveau du tronçon 26 par l'index.

L'opérateur saisit par ailleurs la pince dans sa main droite. L'aiguille est directement visible par l'opérateur.

L'opérateur saisit l'aiguille piquée dans le support 42 qui lui fait directement face au moyen de la pince.

En effectuant un mouvement similaire que celui décrit dans le cadre de la première utilisation décrite précédemment, la portion de fil non-enroulée se tend progressivement entre la première extrémité 220 et les mâchoires de la pince de façon à ce que cette portion de fil non enroulée soit localisée dans le demi-plan de l'espace défini par le côté 12 de l'emballage, et décrive une droite sensiblement alignée avec l'axe X du premier tronçon (effleurant éventuellement la partie terminale d'un doigt 20 du deuxième tronçon 24 le plus proche de l'extrémité 240).

A fur et à mesure que l'opérateur continue à écarter ses deux mains l'une de l'autre, le fil de suture en cours de dévidage va alors se rapprocher, au niveau de l'extrémité 22, du bord extérieur 32 jusqu'à toucher ce dernier, sans toutefois imposer de contrainte mécanique trop importante sur le fil.

L'opérateur souhaitant extraire le fil de suture de la base 1 pourra donc choisir la première ou la deuxième utilisation à sa convenance, sans que le fil ne subisse de frottements supplémentaires dans la première utilisation, par rapport à la deuxième utilisation.

La base 1 définit donc une partie ambidextre d'emballage pour fil de suture.

La longueur L2 du tronçon 22 selon l'axe longitudinal X, depuis lequel s'échappe la portion du fil de suture non enroulée est de préférence choisie plus grande que la longueur L4 du tronçon 24 de façon à offrir à l'opérateur une plus grande surface de préhension sur la base 1 et ainsi améliorer le confort d'utilisation de l'emballage pour extraire le fil de suture qu'il contient. La longueur L2 du tronçon 22 peut par exemple être 2 à 5 fois plus grande que la longueur L4 du tronçon 24.

La partie 50 de l'ouverture traversante 5 localisée dans la zone de parcage 4 s'étend parallèlement à l'axe longitudinal X sur une longueur maximale L5, et la partie 50' de l'ouverture traversante localisée entre les deux extrémités 220, 240 s'étend selon l'axe longitudinal X sur une longueur moyenne L5' qui est inférieure à la longueur L5. Une telle conformation permet d'avoir une ouverture traversante 5 relativement grande dans la zone de parcage, de façon à faciliter l'accès à l'aiguille depuis le côté 14 de l'emballage, et relativement étroite entre les deux extrémités 220 et 240 en regard, ce qui permet d'éviter que la ou les portions de fil enroulé visibles entre les deux extrémités 220 et 240 ne soient trop longues et risquent dès lors de glisser dans la zone de parcage, gênant ainsi l'accès à l'aiguille depuis le côté 14 de la base 1.

La longueur moyenne L5 peut par exemple être 2 à 4 fois plus grande que la longueur L5'.

La longueur L5' de l'ouverture traversante entre les deux extrémités 220 et 240 est de préférence comprise entre 5 mm et 20 mm, de façon à autoriser une extraction selon un axe de déviation limitée tout en évitant que le fil de suture s'échappant depuis l'extrémité 220 ne frotte trop fortement sur l'extrémité 240 au cours de l'extraction ou bien sur l'étiquette cartonnée lorsque celle-ci est accrochée aux moyens d'accrochage 28.

Par ailleurs, on notera qu'avoir un tronçon 22 depuis lequel s'échappe la portion de fil non enroulée qui est rectiligne permet d'inciter naturellement l'opérateur soucieux de ne pas abîmer le fil de suture à opérer un mouvement d'extraction sensiblement selon l'axe longitudinal X correspondant.

Les deux bords de jonction 222 et 242 sont de mêmes largeurs suivant la direction transversale Y.

Par ailleurs, les bords 32 et 34 de la paroi extérieure 3 en les extrémités 22 et 24 en regard sont également de même hauteur selon une direction normale au plan de la base 1.

On notera également que le caractère rectiligne de chaque bord de jonction 222, 242 facilite le glissement du fil de suture en train d'être extrait depuis la zone de parcage 4 vers le bord 32, 34 correspondant.

La base support 1 est de préférence réalisée d'une seule pièce par exemple par moulage.

La base 1 est préférentiellement constituée d'un matériau rigide permettant d'améliorer le confort de préhension de celui-ci par rapport à un cartonnage, et/ou imperméable aux fluides, afin de raccourcir son séchage au cours de sa fabrication. La rigidité de ce matériau est choisie de façon à permettre une levée des doigts sans observer de déformation résiduelle importante.

La base 1 peut présenter de préférence une longueur maximale (mesurée parallèlement à l'axe longitudinal X) comprise entre 70 et 200 mm, préférentiellement 90 mm. La base 1 peut présenter en outre une largeur (mesurée suivant l'axe transversal Y) comprise entre 30 et 70 mm, préférentiellement 38 mm.

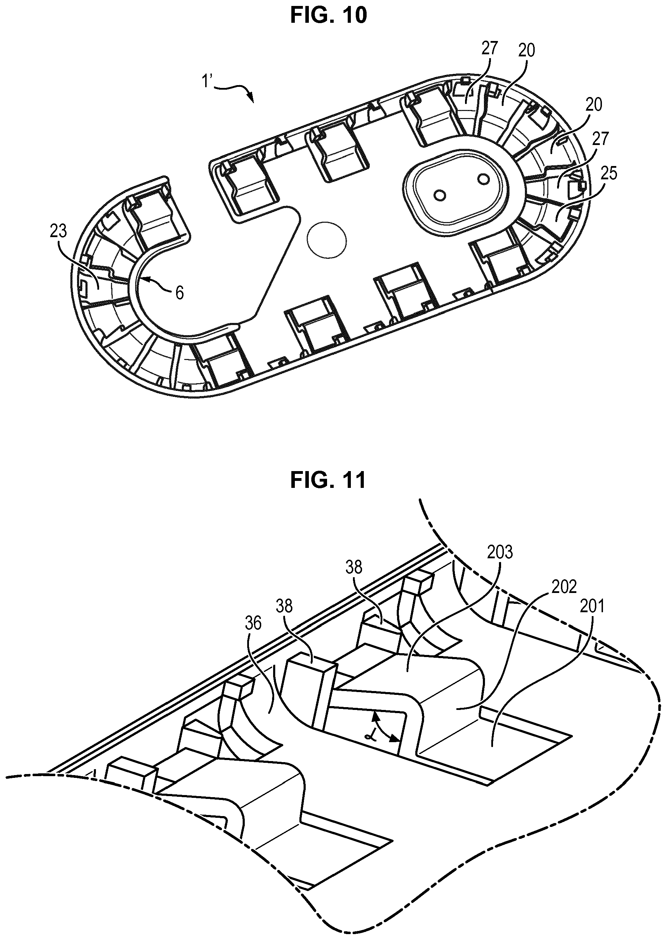

Un deuxième mode de réalisation de la base support, référencé 1' sur les figures 10 à 12, comprend les caractéristiques suivantes en plus de celles de la base 1 déjà décrite.

En référence à la figure 10, les moyens de retenue intérieurs de la base 1' comprennent, sus des doigts de retenue 20 déjà discutés, une pluralité de nervures 27 faisant saillie depuis le côté 12, chaque nervure 27 étant localisée entre deux doigts 20 adjacents de façon à compléter la trajectoire générale déjà définie par les parties 202 de ces doigts 20.

Les nervures 27 sont de préférences localisées entre les doigts définissants les tronçons courbes 23 et 25 de façon à ce que le fil ne force pas sur les doigts 20 lors de son dévidage.

La base 1' comporte en outre une nervure de rigidification 6 s'étendant transversalement au côté 12, depuis la portion 53 définissant un bord de l'ouverture traversante 5. Une telle nervure 6 permet d'augmenter la rigidité du tronçon 23 malgré la présence de l'ouverture 5, et permet aussi de faciliter le remplissage du moule utilisé pour la fabrication de la base support 1'.

Le nombre de doigts peut être réduit sur les tronçons rectilignes 22, 24 et 26 par rapport aux tronçons courbes 23 et 25.

En référence à la figure 11, la paroi périphérique 3 de la base 1' présente une surface interne donnant dans le canal de retenue 2 ayant un profil arrondi à rayon progressif 36 par rapport au côté 12.

En particulier, le bord extérieur 32 et le bord de jonction 222 ne forment pas un « L » mais forment sensiblement un « C » grâce à ce rayon progressif 36 qui interconnecte les deux bords 32 et 222. La paire de bords 34 et 242 présentent une structure similaire.

Dans la base 1', l'espace vide laissé entre la partie terminale de chaque doigt 20 et la surface interne de la paroi périphérique 3 en regard est agrandi par rapport à la base 1 pour faciliter l'injection au cours du moulage de la pièce. Toutefois, avec une telle modification, les doigts 20 ont tendance à moins retenir le fil de suture.

Il est donc prévu des pions 38 faisant saillie depuis la surface interne de la paroi périphérique 3, transversalement au côté 12 (et donc transversalement à la trajectoire d'enroulement du fil). Ces pions permettent d'écarter le fil de la surface interne de la paroi 3 et donc de l'espace vide laissé au-dessus.

Chaque doigt 20 de la base 1' comprend, tout comme la base 1, les parties 201, 202 et 203. Toutefois, la partie 203 terminale ne s'étend pas parallèlement au côté 12 dans la base 1', mais en biais. On peut par exemple prévoir un angle α entre la partie intermédiaire 201 et la partie terminale 203 de valeur comprise entre 45° et 90°. Ceci permet de compenser une déformation résiduelle après la levée des doigts 20 et augmente la robustesse du moule d'injection pour la fabrication de la base 1'.

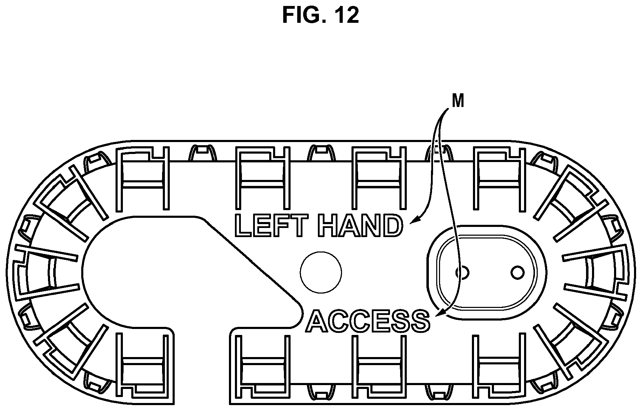

En référence à la figure 12, le côté 14 comprend une inscription M de préférence manuelle. Ici l'inscription est « accès main gauche » (« left hand access ») indiquant que ce côté 14 offre à l'utilisateur un meilleur accès si celui-ci est gaucher et qu'il extrait le fil alors que ce côté 14 lui fait face. Cette inscription est par exemple formée par moulage de caractère à la surface du côté 14, qui offre une plus grande surface d'inscription que le côté 12 opposé. Une inscription peut également être écrite sur l'étiquette cartonnée positionnée au-dessus du côté 12.

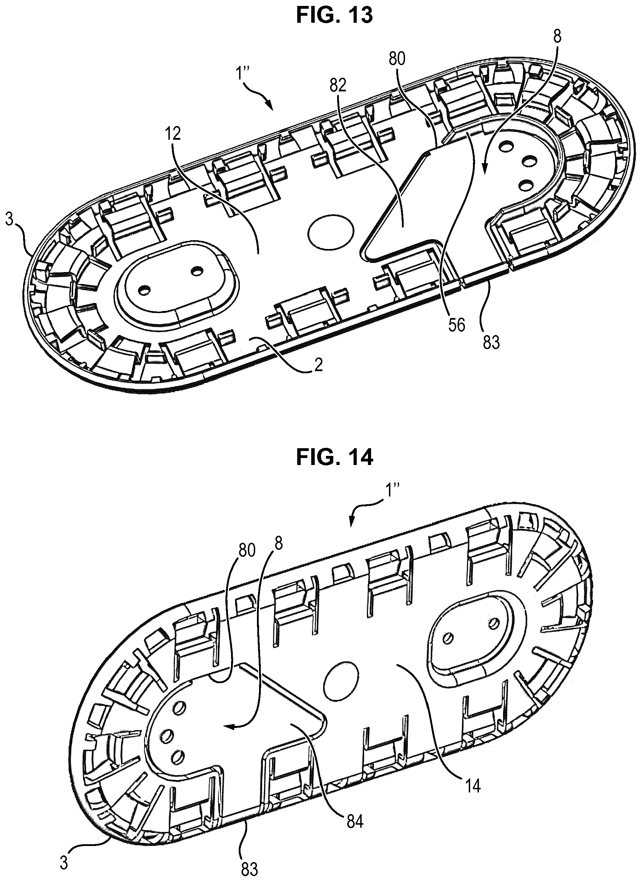

Un troisième mode de réalisation de la base support, référencé 1", est illustré sur les **figures 13 à 15****.**

La base 1" selon ce troisième mode de réalisation se distingue des deux modes de réalisation précédemment décrits par le fait qu'elle comprend en outre un volet 8 mobile entre une position ouverte pour dévoiler l'ouverture traversante 5 entre ses deux côtés 12 et 14, et une position fermée pour boucher au moins en partie l'ouverture traversante 5.

Le volet 8 mobile présente un premier côté 82 formant une portion du côté 12 de la base 1", et un deuxième côté 84 opposé à son premier coté 82 formant une portion du côté 14 de la base 1", lorsque le volet 8 mobile est positionné dans sa position fermée.

La base 1" comprend des moyens d'articulation pour relier le volet 8 au reste du corps de la base 1".

Les moyens d'articulations comprennent une extrémité de liaison 80 du volet mobile 8, cette extrémité étant montée pivotante au reste du corps de la base 1". Le reste du corps de la base 1" comprend notamment la paroi périphérique 3 et le canal de retenue 2.

Cette extrémité 80 définit une ligne de pliure d'axe parallèle à l'axe longitudinal X de la base 1".

L'extrémité de liaison est par exemple connectée au bord rectiligne 56 de l'ouverture traversante longeant le tronçon 26 (ce bord 56 est un bord libre dans les modes de réalisation déjà décrits).

Par ailleurs, le volet 8 mobile présente un bord libre 83, opposé à l'extrémité de liaison 80, et qui est agencé pour être aligné avec la paroi périphérique 3 lorsque le volet 8 mobile est positionné dans sa position fermée. En d'autres termes, le bord libre 83 du volet 8 complète le bord libre formé par la paroi périphérique 3, de sorte à définir avec celle-ci un périmètre fermé de la base 1" lorsque le volet 8 mobile est positionné dans sa position fermée.

Dans une variante du volet mobile 8, le bord libre 83 n'est pas aligné avec la paroi périphérique 3, mais dépasse vers l'extérieur de la base 1" par rapport à la paroi périphérique 3, dans le plan défini par ses deux côtés 12 et 14. Ceci améliore la facilité de préhension du bord libre 83 avec un doigt d'une main tenant par ailleurs le base 1" par un autre partie telle que la paroi périphérique 3.

De la sorte, lorsque le volet 8 est déplacé dans sa position ouverte par pivotement autour de l'extrémité de liaison 80, la partie 50' qui s'étend dans la zone périphérique de façon à interrompre localement le canal de retenue 2 et couper la trajectoire d'enroulement du canal 2 est entièrement dévoilée.

Le volet 8 mobile peut présenter par ailleurs une forme sensiblement complémentaire de l'ouverture traversante 5, de façon à la boucher entièrement lorsqu'il est positionné dans sa position fermée. Il peut être néanmoins prévu un écart résiduel entre les bords de l'ouverture traversante 51, 52, 53, 54 et le volet 8 mobile dans sa position fermée (écart compris entre 0,5 et 3 millimètres, préférentiellement 0,7 millimètres).

Lorsqu'il est complémentaire du contour de l'ouverture traversante 5, le volet 8 comporte une partie logée, en position de repos fermée, dans la partie 50' de l'ouverture qui s'étend dans la zone périphérique de façon à interrompre localement le canal de retenue 2 et couper la trajectoire d'enroulement du canal 2. L'extrémité de liaison 80 est formée sur le bord du volet 8 opposé au bord du volet 8 qui affleure la périphérie extérieure de la base 1", en positon de repos fermée.

L'extrémité de liaison 80, de préférence venue de matière sur la base 1" et le volet 8, peut être formée par rétrécissement local d'épaisseur par rapport à l'épaisseur de la base 1" et du volet 8. Un tel rétrécissement facilite l'ouverture du volet 8 par pliage au niveau de la liaison 80.

Lorsque le volet 8 est positionné dans sa position ouverte, un opérateur peut avoir accès, en passant un instrument de préhension dans l'ouverture traversante 5 depuis le côté 14 de la base 1", à la portion d'extrémité libre du fil de suture destinée à être saisie pour son extraction, selon une utilisation similaire à celle décrite en relation avec les figures 6 et 7.

Lorsque le volet 8 est positionné dans sa position fermée, le volet 8 bouche l'ouverture traversante 5. Le volet 8 forme alors un écran de protection entre cette portion d'extrémité du fil de suture à saisir, localisée du côté 12 de la base, et tout objet situé au-delà de l'autre côté 14 de la base 1 ".

Un tel volet 8 permet dans sa position fermée de limiter l'espace de liberté de l'extrémité du fil de suture destinée à être saisie pour son extraction.

Le volet 8 est particulièrement avantageux lorsque l'extrémité libre du fil de suture à saisir est pourvue d'une aiguille. En effet, l'aiguille, qui présente une structure rigide, risque d'endommager la base 1" elle-même et/ou un emballage dans lequel la base 1" est conditionnée, par frottement ou suite à son détachement de son support 42, par exemple au cours du transport de la base 1".

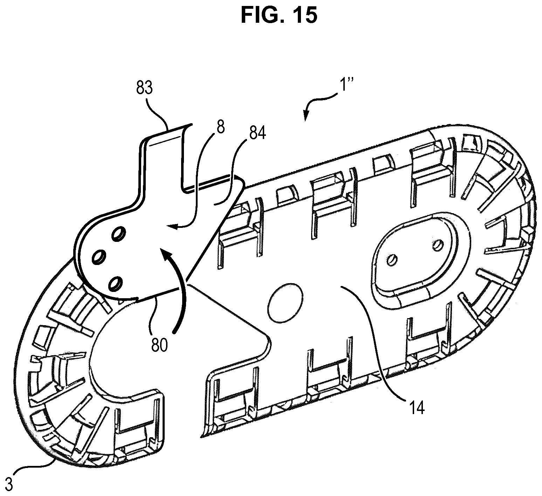

Le volet 8 mobile est agencé pour faire saillie, dans sa position ouverte, depuis le côté 14 de la base opposé au côté 12 sur lequel le canal de retenue 2 est formé, comme il est représenté sur la figure 15.

De préférence, le volet 8 mobile et le reste du corps de la base 1" font partie d'une seule pièce ; le volet 8 mobile étant alors une partie venue de matière sur la base 1", pliable de la base par rapport au reste de son corps (notamment par rapport au canal de retenue 2 et à la zone de parcage 4).

Par ailleurs, le volet 8 mobile peut être configuré de sorte à occuper au repos sa position fermée, et ainsi être sollicité naturellement par retour élastique vers cette position fermée, après avoir été déplacé par sollicitation extérieure, vers la position ouverte.

Un fil de suture enroulé dans la base 1" peut être extrait hors de celle-ci au moyen des étapes déjà décrites.

Toutefois, avant de faire passer un instrument de préhension à travers l'ouverture traversante selon la première utilisation déjà décrite en relation avec les figures 6 à 8, une étape supplémentaire de dévoilement de l'ouverture traversante par déplacement du volet 8 dans sa position ouverte, en saillie du côté 14, est mise en oeuvre.

L'opérateur peut maintenir le volet 8 dans sa position ouverte à l'aide de la main qu'il utilise pour tenir le corps de la base pendant le dévidage du fil de suture. L'utilisateur peut par exemple pincer la base en positionnant son pouce contre le côté 14, en une position telle que le pouce est partiellement localisé entre le volet 8 en saillie et l'ouverture traversante.

En variante, la liaison 80 est adaptée pour que le volet mobile 8 puisse rester dans sa position ouverte naturellement, sans sollicitation extérieure. Ceci permet à l'opérateur se saisissant de la base 1" de ne pas avoir à maintenir manuellement le volet 8 en position ouverte, et ainsi d'améliorer le confort de saisie de la base 1" par cet opérateur.

La base support selon l'invention ne se limite évidemment pas aux modes de réalisation illustrés sur les figures mais peut faire l'objet d'autres variantes dont une liste non limitative est donnée ci-après.

Il n'est pas obligatoire que l'ouverture traversante coupe le canal de retenue sur une section entière celui-ci, jusqu'à atteindre et interrompre la paroi extérieure 3, comme il a été décrit dans le mode de réalisation illustré. Il suffit en effet que l'ouverture traversante se prolonge depuis la zone de parcage de façon à couper au moins partiellement une section du canal de retenue, de sorte à permettre une déviation limitée du fil de suture lorsque celui-ci est tiré par l'opérateur dans l'une au l'autre des deux utilisations décrites.

Le canal de retenue 2 peut être modifié pour définir de façon générale une trajectoire d'enroulement ellipsoïde, allant d'une forme circulaire à une forme en hippodrome comme représentée dans le mode de réalisation illustré.

Les tronçons 22 et 24 peuvent ainsi être courbes et non rectilignes.

Le canal de retenue 2 peut être réalisé autrement qu'au moyen de doigts 20 pivotants. Par exemple, la base 1 peut être constituée de deux pièces formant après assemblage le canal de retenue 2.

La portion du fil de suture non enroulée peut en outre s'échapper du tronçon 24 et non du tronçon 22.

Les extrémités 220 et 240 en regard peuvent présenter des dimensions identiques ou différentes. Par exemple, les bords de jonction 222 et 242 peuvent être de largeurs respectives différentes.

## Revendications

1. Base support (1") pour fil de suture, la base support (1") présentant deux côtés opposés (12, 14) et comprenant sur un (12) de ses côtés :
- un canal de retenue (2) d'un fil de suture dans une position enroulée,
- une zone de parcage (4) d'une extrémité du fil de suture, la zone de parcage (4) étant entourée par le canal de retenue (2),
la base support (1") comprenant une ouverture traversante (5) entre ses deux côtés (12, 14), l'ouverture traversante (5) s'étendant dans la zone de parcage (4) et se prolongeant depuis la zone de parcage (4) de façon à couper le canal de retenue (2), ladite base support étant **caractérisée en ce qu'**elle comprend en outre un volet mobile (8) entre une position ouverte pour dévoiler l'ouverture traversante (5) entre ses deux côtés (12, 14), et une position fermée pour boucher au moins en partie l'ouverture traversante (5), le volet mobile (8) étant configuré pour faire saillie, dans sa position ouverte, depuis le côté (14) de la base (1") qui est opposé au côté (12) sur lequel le canal de retenue (2) est formé.

2. Base support (1") selon la revendication 1, dans laquelle, le canal de retenue (2) est délimité extérieurement par une paroi périphérique (3) constituant un bord libre de la base support (1"), et l'ouverture traversante (5) se prolonge jusqu'à couper également la paroi périphérique (3).

3. Base support (1") selon l'une des revendications 1 et 2, comprenant une nervure de rigidification (6) faisant saillie depuis au moins un des deux côtés opposés (12, 14), la nervure de rigidification (6) s'étendant le long d'un bord de l'ouverture traversante (5).

4. Base support (1") selon la revendication 3, dans laquelle la nervure de rigidification s'étend entre l'ouverture traversante (5) et un tronçon (23) du canal de retenue (2) présentant globalement une forme en C.

5. Base support (1") selon l'une des revendications 1 à 4, dans laquelle le canal de retenue (2) comprend un premier tronçon (22) dont une première extrémité (220) débouche dans l'ouverture traversante (5), l'extrémité du fil localisée dans la zone centrale de parcage s'échappant du canal de retenue (2) par la première extrémité (220).

6. Base support (1") selon la revendication 5, dans laquelle le premier tronçon (22) est rectiligne.

7. Base support (1") selon l'une des revendications 5 et 6, dans laquelle le premier tronçon (22) s'étend selon un axe longitudinal (X) et la première extrémité (220) présente un bord de jonction (222) rectiligne reliant les deux côtés de la base, le bord de jonction (222) s'étendant perpendiculairement à l'axe longitudinal (X).

8. Base support (1") selon l'une des revendications 5 à 7, dans laquelle le canal de retenue (2) comprend un deuxième tronçon (24) dont une deuxième extrémité (240) en regard de la première extrémité (220) débouche également dans l'ouverture traversante (5), le deuxième tronçon s'étendant suivant le même axe longitudinal (X).

9. Base support (1") selon la revendication 8, dans laquelle la première extrémité (220) et la deuxième extrémité (240) sont de mêmes largeurs entre la zone de parcage (4) et la paroi périphérique de la base (3).

10. Base support (1") selon l'une des revendications 8 et 9, dans laquelle l'ouverture traversante (5) s'étend entre les deux extrémités de tronçon en regard (220, 240) sur une première longueur (L5') suivant l'axe longitudinal (X), et s'étend dans la zone de parcage (4) sur une deuxième longueur (L5) parallèle et supérieure à la première longueur (L5').

11. Base support (1") selon l'une des revendications 5 à 10, dans laquelle le premier tronçon (22) présente une longueur (L2) suivant l'axe longitudinal supérieure à celle (L4) du deuxième tronçon (24).

12. Base support (1") selon l'une des revendications précédentes, dans laquelle le canal de retenue (2) présente une forme adaptée pour contenir un fil de suture enroulé selon une trajectoire sensiblement ellipsoïde.

13. Base support (1") selon l'une des revendications précédentes, réalisé en un matériau rigide et/ou imperméable.

14. Base support (1") selon l'une des revendications précédentes, dans laquelle le volet mobile (8) présente une extrémité de liaison (80) montée pivotante à une partie de la base (1") solidaire de la paroi périphérique (3), et un bord libre (83) opposé à l'extrémité de liaison (80) agencé pour être aligné avec ou dépassant de la paroi périphérique (3) lorsque le volet mobile (8) est positionné dans sa position fermée.

15. Base support (1") selon l'une des revendications précédentes, dans lequel le volet mobile (8) présente deux côtés (82, 84) opposés formant chacun une portion des côtés (12, 14) opposés de la base (1") lorsque le volet mobile est positionné dans sa position fermée.

16. Procédé d'extraction d'un fil de suture enroulé dans le canal de retenue (2) d'une base support (1") selon l'une des revendications précédentes, le procédé comprenant les étapes suivantes :
- insertion d'un moyen de préhension à travers l'ouverture traversante (5) par le côté (14) de la base opposé au côté (12) sur lequel le canal de retenue (2) est formé,
- saisie de l'extrémité du fil de suture parquée dans la zone de parcage (4) à l'aide du moyen de préhension passé à travers l'ouverture traversante,
- éloignement mutuel de la base (1") et de l'extrémité du fil de suture saisie par le moyen de préhension.

17. Procédé d'extraction selon la revendication précédente, comprenant en outre un déplacement du volet mobile (8) depuis sa position fermée dans sa position ouverte, avant l'étape d'insertion.

## Patentansprüche

1. Trägerbasis (1") für einen Nahtfaden, wobei die Trägerbasis (1") zwei gegenüberliegende Seiten (12, 14) aufweist und auf einer (12) seiner Seiten umfasst:
- einen Kanal zum Halten (2) eines Nahtfadens in einer aufgewickelten Stellung,
- eine Zone zum Parken (4) eines Endes des Nahtfadens, wobei die Parkzone (4) vom Haltekanal (2) umgeben ist,
wobei die Trägerbasis (1") zwischen ihren zwei Seiten (12, 14) eine Durchgangsöffnung (5) umfasst, wobei sich die Durchgangsöffnung (5) in der Parkzone (4) erstreckt und sich von der Parkzone (4) aus verlängert, sodass sie den Haltekanal (2) schneidet, wobei die Trägerbasis **dadurch gekennzeichnet ist, dass** sie weiter eine zwischen einer offenen Stellung zum Freilegen der Durchgangsöffnung (5) zwischen ihren zwei Seiten (12, 14), und einer geschlossenen Stellung zum mindestens teilweisen Versperren der Durchgangsöffnung (5) bewegliche Klappe (8) umfasst, wobei die bewegliche Klappe (8) dafür ausgestaltet ist, in ihrer offenen Stellung von der Seite (14) der Basis (1") aus vorzuspringen, die der Seite (12) gegenüberliegt, auf welcher der Haltekanal (2) gebildet ist.

2. Trägerbasis (1") nach Anspruch 1, wobei der Haltekanal (2) außen von einer Umfangswand (3) begrenzt wird, die eine freie Kante der Trägerbasis (1") bildet, und sich die Durchgangsöffnung (5) verlängert, bis sie ebenfalls die Umfangswand (3) schneidet.

3. Trägerbasis (1") nach einem der Ansprüche 1 und 2, die eine Versteifungsrippe (6) umfasst, welche von mindestens einer der zwei gegenüberliegenden Seiten (12, 14) aus vorspringt, wobei sich die Versteifungsrippe (6) entlang einer Kante der Durchgangsöffnung (5) erstreckt.

4. Trägerbasis (1") nach Anspruch 3, wobei sich die Versteifungsrippe zwischen der Durchgangsöffnung (5) und einem Teilstück (23) des Haltekanals (2) erstreckt, das allgemeine eine C-Form aufweist.

5. Trägerbasis (1") nach einem der Ansprüche 1 bis 4, wobei der Haltekanal (2) ein erstes Teilstück (22) umfasst, von dem ein erstes Ende (220) in die Durchgangsöffnung (5) mündet, wobei das Ende des Fadens, das in der zentralen Parkzone liegt, durch das erste Ende (220) aus dem Haltekanal (2) austritt.

6. Trägerbasis (1") nach Anspruch 5, wobei das erste Teilstück (22) gerade ist.

7. Trägerbasis (1") nach einem der Ansprüche 5 und 6, wobei sich das erste Teilstück (22) entlang einer Längsachse (X) erstreckt, und das erste Ende (220) eine gerade Verbindungskante (222) aufweist, die die zwei Seiten der Basis verbindet, wobei sich die Verbindungskante (222) senkrecht zur Längsachse (X) erstreckt.

8. Trägerbasis (1") nach einem der Ansprüche 5 bis 7, wobei der Haltekanal (2) ein zweites Teilstück (24) umfasst, von dem ein zweites Ende (240), das dem ersten Ende (220) zugewandt ist, ebenfalls in die Durchgangsöffnung (5) mündet, wobei sich das zweite Teilstück entlang derselben Längsachse (X) erstreckt.

9. Trägerbasis (1") nach Anspruch 8, wobei das erste Ende (220) und das zweite Ende (240) zwischen der Parkzone (4) und der Umfangswand der Basis (3) von gleichen Breiten sind.

10. Trägerbasis (1") nach einem der Ansprüche 8 und 9, wobei sich die Durchgangsöffnung (5) zwischen den zwei zugewandten Teilstückenden (220, 240) auf einer ersten Länge (L5') entlang der Längsachse (X) erstreckt, und sich in der Parkzone (4) auf einer zweiten Länge (L5) erstreckt, die parallel zur und größer als die erste Länge (L5') ist.

11. Trägerbasis (1") nach einem der Ansprüche 5 bis 10, wobei das erste Teilstück (22) eine Länge (L2) entlang der Längsachse aufweist, die größer als diejenige (L4) des zweiten Teilstücks (24) ist.

12. Trägerbasis (1") nach einem der vorstehenden Ansprüche, wobei der Haltekanal (2) eine Form aufweist, die dafür angepasst ist, einen Nahtfaden zu enthalten, der entlang einer im Wesentlichen ellipsoiden Bahn aufgewickelt ist.

13. Trägerbasis (1") nach einem der vorstehenden Ansprüche, die aus einem starren und/oder undurchlässigen Material ausgeführt ist.

14. Trägerbasis (1") nach einem der vorstehenden Ansprüche, wobei die bewegliche Klappe (8) ein Verbindungsende (80) aufweist, das an einem fest mit der Umfangswand (3) verbundenen Teil der Basis (1") schwenkbar montiert ist, und eine dem Verbindungsende (80) gegenüberliegende freie Kante (83), die dafür eingerichtet ist, mit der Umfangswand (3) ausgerichtet zu sein oder über dieselbe hinauszugehen, wenn die bewegliche Klappe (8) in ihrer geschlossenen Stellung positioniert ist.

15. Trägerbasis (1") nach einem der vorstehenden Ansprüche, wobei die bewegliche Klappe (8) zwei gegenüberliegende Seiten (82, 84) aufweist, die jede einen Abschnitt der gegenüberliegenden Seiten (12, 14) der Basis (1") bilden, wenn die bewegliche Klappe in ihrer geschlossenen Stellung positioniert ist.

16. Verfahren zum Herausziehen eines Nahtfadens, der im Haltekanal (2) einer Trägerbasis (1") nach einem der vorstehenden Ansprüche aufgewickelt ist, wobei das Verfahren die folgenden Schritte umfasst:
- Einführen eines Griffmittels durch die Durchgangsöffnung (5) hindurch von der Seite (14) der Basis her, die der Seite (12) gegenüberliegt, auf der der Haltekanal (2) gebildet ist,
- Greifen des in der Parkzone (4) geparkten Endes des Nahtfadens mithilfe des durch die Durchgangsöffnung hindurchgeführten Griffmittels,
- Entfernen der Basis (1") und des mit dem Griffmittel gegriffenen Endes des Nahtfadens voneinander.

17. Herausziehverfahren nach dem vorstehenden Anspruch, das vor dem Schritt des Einführens weiter ein Bewegen der beweglichen Klappe (8) von ihrer geschlossenen Stellung aus in ihre offene Stellung umfasst.

## Claims

1. A support base (1") for a suture thread, the support base (1") having two opposite sides (12, 14) and comprising on one (12) of its sides:
- a storage channel (2) for a suture thread in a wound position,
- a holding zone (4) for an end of the suture thread, the holding zone (4) being surrounded by the storage channel (2),
the support base (1") comprising a through opening (5) between its two sides (12, 14), the through opening (5) extending into the holding zone (4) and continuing from the holding zone (4) so as to intersect the storage channel (2), said support base being **characterized in that** it also comprises a shutter (8) movable between an open position for uncovering the through opening (5) between its two sides (12, 14) and a closed position for blocking, at least partially, the through opening (5), the movable shutter (8) being configured to project, in its open position, from the side (14) of the base (1") which is opposite to the side (12) on which the storage channel (2) is formed.

2. The support base (1") according to claim 1 wherein the storage channel (2) is delimited externally by a peripheral wall (3) constituting a free edge of the support base (1"), and the through opening (5) continues until it also intersects the peripheral wall (3).

3. The support base (1") according to one of claims 1 and 2, comprising a stiffening rib (6) projecting from at least one of the two opposite sides (12, 14), the stiffening rib (6) extending along an edge of the through opening (5).

4. The support base (1") according to claim 3, wherein the stiffening rib extends between the through opening (5) and a segment (23) of the storage channel (2) having the overall shape of a C.

5. The support base (1") according to one of claims 1 to 4, wherein the storage channel (2) comprises a first segment (22) of which a first end (220) leads into the through opening (5), the end of the thread located in the central holding zone leaving the storage channel (2) through the first end (220).

6. The support base (1") according to claim 5, wherein the first segment (22) is rectilinear.

7. The support base (1") according to one of claims 5 and 6, wherein the first segment (22) extends along a longitudinal axis (X) and the first end (220) has a rectilinear junction edge (222) connecting the two sides of the base, the junction edge (222) extending perpendicularly to the longitudinal axis (X).

8. The support base (1") according to one of claims 5 to 7, wherein the storage channel (2) comprises a second segment (24) of which a second end (240) facing the first end (220) also leads into the through opening (5), the second segment extending along the same longitudinal axis (X).

9. The support base (1") according to claim 8, wherein the first end (220) and the second end (240) have the same length between the holding zone (4) and the peripheral wall (3) of the base.

10. The support base (1") according to one of claims 8 and 9, wherein the through opening (5) extends between the two facing ends of the segment (220, 240) over a first length (L5') along the longitudinal axis (X) and extends into the holding zone (4) over a second length (L5) parallel to and greater than the first length (L5').

11. The support base (1") according to one of claims 5 to 10, wherein the first segment (22) has a length (L2) along the longitudinal axis greater than that (L4) of the second segment (24).

12. The support base (1") according to one of the foregoing claims, wherein the storage channel (2) has a shape suited to hold the suture thread wound along a substantially elliptical trajectory.

13. The support base (1") according to one of the foregoing claims, made of a rigid and/or impermeable material.

14. The support base (1") according to one of the foregoing claims, wherein the movable shutter (8) has a connection end (80) mounted pivotally on a portion of the base (1") integral with the peripheral wall (3), and a free edge (83) opposite to the connection end (80) positioned to be aligned with or extend beyond the peripheral wall (3) when the movable shutter (8) is positioned in its closed position.

15. The support base (1") according to one of the foregoing claims, wherein the movable shutter (8) has two opposite sides (82, 84) each forming a portion of the opposite sides (12, 14) of the base (1") when the movable shutter is positioned in its closed position.

16. A method of extracting a suture thread wound into the storage channel (2) of a support base (1") according to one of the foregoing claims, the method comprising the following steps:
- insertion of a grasping means through the through opening (5) by the side (14) of the base opposite to the side (12) on which the storage channel (2) is formed,
- seizure of the end of the suture thread held in the holding zone (4) by means of the grasping means passed through the through opening,
- mutual separation of the base (1") and of the end of the suture thread seized by the grasping means.

17. The extraction method according to the foregoing claim, also comprising a movement of the movable shutter (8) from its closed position into its open position, before the insertion step.
